# EUROPEAN PATENT APPLICATION

(11) **EP 4 029 531 A1**
(43) Date of publication of application: **20.07.2022**
(21) Application number: 21151331.2
(22) Date of filing: 13.01.2021
(51) Int. Cl.: A61L 2/10, E05B 1/00, G02B 6/00

(54) **STERILIZATION SYSTEM**

(71) Applicant: Koninklijke Philips N.V., 5656 AG Eindhoven (NL)
(72) Inventor: Salters, Bart Andre, 5656 AE Eindhoven (NL); NIESSEN, Eduard Matheus Johannes, 5656 AE Eindhoven (NL); Martens, Peter, 5656 AE Eindhoven (NL); Hietbrink, Roelant Boudewijn, 5656 AE Eindhoven (NL)
(74) Representative: Philips Intellectual Property & Standards

(57) **Abstract**

A system is provided for sterilizing an actuation surface which is for contact by a user to implement an actuation. A cover part includes the actuation surface and is for transferring actuation to a base. The cover part comprises a waveguide which is transparent to sterilizing light so that sterilizing light can be transmitted from a sterilizing light source to the actuation surface. The system has a first portion which is removable from the base for replacement and includes and least the cover part. A second portion is intended for continued use when the first portion is replaced. A coupling between the first and second portions implements an electrical and optical coupling.

## Description

### FIELD OF THE INVENTION

This invention relates to a system for sterilizing surfaces, in particular actuation surfaces, by which is meant surfaces which are contacted by hand by a person to implement a function of a device or object.

### BACKGROUND OF THE INVENTION

There are numerous objects in daily life which are meant to be used as an actuator to be manipulated by hand, to achieve a certain goal. Examples are door knobs or levers, toilet seats, (elevator) buttons, touchscreens, ATM machines, payment terminals, vending machines, as well as more common tools such as hammers and screwdrivers.

In many cases, these actuators are used in public spaces, and as a consequence, they might be used and hence touched by numerous people in a short span of time. This provides an excellent way of spreading diseases, if contagious people handle such surfaces.

It is highly desirable to come up with a method of disinfecting such surfaces, especially those in public spaces.

Lighting systems for application to a surface to provide sterilization at the surface, for example to prevent biofouling, are known. In particular, it is well-known that most micro-organisms are killed, rendered inactive or unable to reproduce with sufficient UV radiation. This effect is mainly governed by the total dose of UV radiation. A typical dose to kill 90% of a certain micro-organism is 10mWh per square meter.

Ultraviolet (UV) is that part of electromagnetic light bounded by the lower wavelength extreme of the visible spectrum and the X-ray radiation band. The spectral range of UV radiation is by definition between 100 and 400 nm and is invisible to human eyes. Using the CIE classification the UV spectrum is subdivided into three bands:
UVA (long-wave) from 315 to 400 nm
UVB (medium- wave) from 280 to 315 nm
UVC (short-wave) from 100 to 280 nm

Various light sources for generating UV are known, such as low-pressure mercury discharge lamps, medium pressure mercury discharge lamps and dielectric barrier discharge lamps.

A preferred option is low cost, low power UV LEDs. LEDs can generally be included in smaller packages and consume less power than other types of light sources. LEDs can be manufactured to emit (UV) light of various desired wavelengths and their operating parameters, most notably the output power, can be controlled to a high degree. A suitable germicidal dose can easily be achieved with existing UV LEDs, for example UVC LEDs.

WO 2018/215272 discloses a system for sterilizing a surface of a waveguide by providing UV radiation into the waveguide, wherein UV radiation exits the waveguide to provide a sterilizing function to the surface of the waveguide. An amount of light is detected which is dependent on the amount of UV radiation which is internally reflected (i.e. which did not exit the waveguide and was not absorbed) and this can be used to determine if the surface has been contacted. The UV radiation may then be reduced or switched off when it is detected that a person is nearby to reduce UV exposure of the person.

WO 2018/215272 relates primarily to the prevention of biofouling of surfaces which are maintained under water, such as the hull of a ship. However, an example given in WO 2018/215272 is a door knob in which UV radiation is used to keep the knob clean, but the radiation is switched off when the door knob is contacted.

In some aspects, this invention relates in particular to surfaces to be sterilized which are meant to be touched by hand, and will hence become get scratched or damaged over time, leading to degraded performance. For example, undesirable out-coupling of light from the waveguide may result even when nothing is touching the light guide. This out-coupling of light risks leakage of the UV light into the environment, which is undesirable at least because it wastes power and it possibly exposes the surroundings (including human beings) to UV radiation. When contact detection is based on measuring light levels, the out-coupling of light resulting from a damaged surface can also make the system less reliable in detecting human contact (touch) events.

The system also needs to be powered to deliver energy to the light source. In existing systems, this means either connections are needed to a mains supply or else a battery is needed, which will then require servicing or replacement.

The invention is directed to one or more of these issues.

### SUMMARY OF THE INVENTION

The invention is defined by the claims.

According to examples in accordance with the invention, there is provided a system for sterilizing an actuation surface which is for contact by a user to implement an actuation, the system comprising:
a cover part including the actuation surface, the cover part being for fitting to a base, the cover part being for transferring actuation to the base through the cover part;
a light source for providing sterilizing light to the actuation surface; and
a controller for controlling the light source,
wherein the cover part comprises a waveguide which is transparent to the sterilizing light, thereby to transmit the sterilizing light from the light source to the actuation surface,
wherein the system comprises a first portion which is removable from the base for replacement and includes at least the cover part, and a second portion which is intended for continued use when the first portion is replaced, and
wherein the system comprises a coupling between the first and second portions which implements an electrical and optical coupling.

In this system, the first portion is primarily an optical part and it is preferably disposable and can easily be replaced. It is for example primarily a passive optical part. The second portion may be considered to be an active part, that includes at least some of the electronic or active optical components, such as the light source (LEDs), a detector, and/or driving electronics. In this way, when the actuation surface becomes damaged it can be replaced simply and with low cost.

However, there is an electrical and optical coupling between the first and second portions, so that both optical and electrical components can be provided in each portion. This gives the flexibility to design optical and electrical components either as replaceable items or as fixed items. At least the cover part is one of the replaceable items.

By fitting the first portion to the second portion, an electrical and optical coupling is implemented so that power can be transferred between the first and second portions and light can be coupled into the waveguide (formed by the removable cover part). The fitting of the first portion to the base also forms a mechanical coupling by which force applied to the cover part is transferred to the base.

The mechanical coupling secures the first portion against rotation and it also sets the correct positional relationship to establish a correct electrical contact.

The system for example further comprises a detector for detecting at least touching of the actuation surface, wherein the controller is for controlling the light source in dependence on at least detected touching. The detector for example comprises a light detector for detecting a light level inside the waveguide, and the controller is adapted to detect touching by detecting a change in the detected light level. For example, the touching alters the refractive index boundary conditions and thus allows more light to escape from the waveguide. In other scenarios, the detector may observe an increase, for example if a highly reflective object contacts the waveguide.

Other ways of detecting touching may be used, for example by detection the resulting actuation rather than the actual contact.

The second (fixed) portion typically comprises the active electronic components, such as the light source. The second (removable) portion will typically have very few, if any, of those. However, a detector may for example be provided in the first portion part as this makes it easier to implement the detection function.

The controller is for example adapted to (immediately) turn off the light source during detected touching. This reduces the exposure of a person to the generated UV radiation.

The light source may then be turned on after the detected touching to provide a sterilizing function.

The controller may be further adapted to detect contamination of the actuation surface by detecting a change in light out-coupled from the waveguide (less than that when the surface is touched), and retain the light source turned on in response to detected contamination. In this way, the light source remains activated if out-coupling is changed (e.g. increased) due to surface contamination.

The rate of change over time is a strong indicator of human touch, versus contamination which builds up more slowly over time. Thus, the detection may be based both on the absolute value of a detected signal as well as the temporal characteristics.

The controller may be further adapted to detect a level of wear and tear of the cover part. For example, scratches may be detected as they also cause an increase in light out-coupling, and again with different temporal characteristics to a hand touching event or contamination.

The optical coupling for example comprises face-to-face contact between a light entry surface of the waveguide and a light emission surface of the light source when the first portion is fully fitted to the base.

The first portion for example has a polygonal central bore for fitting over a corresponding polygonal outer shape of the base, thereby to implement a mechanical coupling. This coupling transfers force applied to the cover part to the base, so that the mechanical actuation takes place. The polygonal central bore is for example square or rectangular.

A rotationally symmetric shape may be used so that there are multiple possible fitting orientations or a non-rotationally symmetric shape may be used so that the first portion can be fitted in only one, correct, orientation.

The system may comprise one or more wires extending along the base and terminating at one or more first terminals at a distal end of the base, and the first portion has a closed end including one or more second terminals for coupling to the first terminals, thereby to implement the electrical coupling when the first portion is fully fitted to the base.

Thus, fitting the first portion to the base results in an electrical connection being formed between one or more terminals at the end of the base and at the closed end of the first portion.

Instead, or as well, there may be one or more first terminals at a proximal end of the base, and the first portion comprises one or more second terminals at a distal end for coupling to the first terminals. Thus, fitting the first portion to the base results in an electrical connection being formed between one or more terminals at the start of the base and at the open end of the first portion.

The terminal arrangement and/or the base and first portion shapes may be designed so that the first portion can connect to the base in only one angular orientation. Alternatively, the terminal arrangement may be central so that the first portion can be mounted over the base in multiple possible angular orientations.

By way of example, the first portion may have a hexagonal cross section, so there are six possible ways of mounting the first portion. One possibility is that only only one of those establishes the required electrical connection, so that the LED is turned on and the system is live. Simply re-attaching the first portion in any of the other five possible ways will turn the system off. This could be a setting where there is no need for the sterilization.

Different rotational positions of the first portion may also be used to implement different functions; for example a double power setting for quick disinfection, or a higher sensitivity to touch (for additional safety); which could be achieved by establishing an electrical connection to a different, more sensitive, detector.

The first portion may comprise a battery in a space between the base and the cover part. In one example, the battery has a hollow core which defines a central bore of the first portion. Thus, the battery is formed in an annular shape around the base. Alternatively, the battery can be provided in a hollow portion of the base.

The system may comprise first and second cover parts of respective first and second first portions, for sterilizing a pair of door handles, wherein the system comprises an electrical connection (e.g. two or more electrical wires for transferring power) between the first and second first portions for passing through a door.

In this way two sterilizing systems may share a power supply and/or a controller, for powering and controlling the sterilizing systems for each door handle.

Thus, there may be only one controller for mounting at the side of only one door handle and there may be only one battery for mounting at the side of only one door handle.

The light source may also be for mounting at the side of only one door handle, and the system then further comprises an optical connection (e.g. a glass fiber) between the first and second first portions for passing through the door. Thus, the light source may be shared between two door handles.

The light source for example comprises a UV light source. It is known that UV light can be used to perform a sterilizing function.

The invention also provides a door handle, comprising:
a base; and
the system defined above defining an actuation surface for transferring actuation to the shaft through the cover part.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an example of a manually operated device, in particular a door handle;
Fig. 2 shows a first example of an arrangement of components for the system of Fig. 1;
Fig. 3 shows a second example of an arrangement of components for the system of Fig. 1;
Fig. 4 shows a third example of an arrangement of components for the system of Fig. 1;
Fig. 5 shows a fourth example of an arrangement of components for the system of Fig. 1;
Fig. 6 shows a first way to implement electrical and optical connections;
Fig. 7 shows a second way to implement electrical and optical connections;
Fig. 8 shows another alternative design in which the battery is defined in a space between the shaft and the cover part; and
Fig. 9 shows an alternative example in which energy is harvested from the normal manual actuation.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for sterilizing an actuation surface which is for contact by a user to implement an actuation. A cover part includes the actuation surface and is for transferring actuation to a base. The cover part comprises a waveguide which is transparent to sterilizing light so that sterilizing light can be transmitted from a sterilizing light source to the actuation surface. The system has a first portion which is removable from the base for replacement and includes at least the cover part. A second portion is intended for continued use when the first portion is replaced. A coupling between the first and second portions implements an electrical and optical coupling. The replaceable part has more than just a passive optical component as a replaceable part. For example the replaceable part may also include a battery, or a sensor or a mirror, etc.

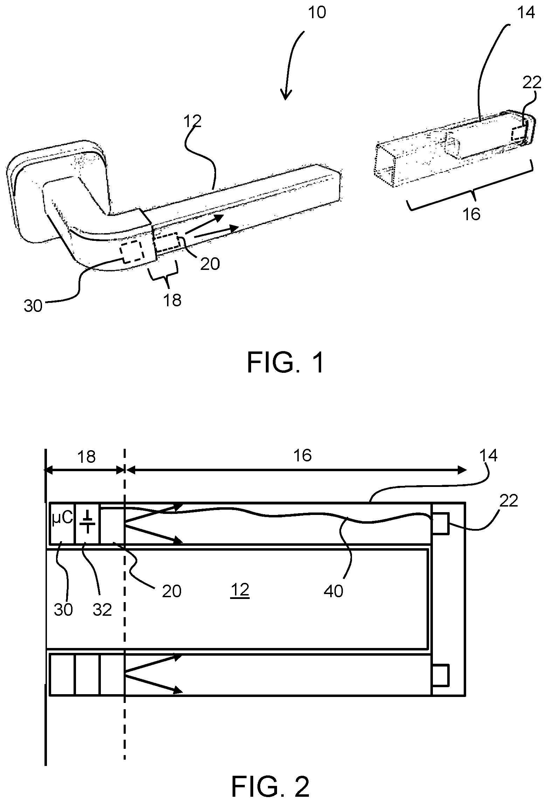

Fig. 1 shows an example of a manually operated device, in particular a door handle 10. The door handle 10 comprises a base 12 to which an external force is to be applied by a user to operate the door handle.

The invention relates generally to any manually operated device. Such devices have in common a surface which is to be handled by a user, wherein a force or just contact with the surface is for implementing operation of the device. In the case of a door handle, a force to the base 12 causes the handle to rotate about a pivot axis and thereby operate a door latch.

The handle does not need to be a door handle for operating a latch. The handle may for example be the handle of any manually operated tool or implement such as a drawer (i.e. without a pivot action).

The same general function is implemented by any manual actuator. For example, for a touch or push button (e.g. in a lift or an ATM machine) there is a base which is the touch or push button. For a touch or push keypad the base is an array of buttons. A force to the button or buttons causes the button or buttons to move (depress) or simply register and thereby actuate a corresponding circuit.

The door handle further comprises a cover part 14 which defines the external contact surface that will be contacted by a user. It covers the base 12 (it is shown removed in Fig. 1). The cover part functions as a waveguide, which distributes light internally by total internal reflection at the outer material-air refractive index boundary. The cover part is for example a glass or quartz cylinder, which is slid over the extending end of the handle. Any suitable material may be used which is transparent to the sterilization light to be used. The refractive index of the solid cover part creates a refractive index difference at the outer surface to implement the required total internal reflection. The resulting external contact surface becomes the actuation surface of the overall device. Thus, the cover part 14 transfers actuation to the base 12 through the cover part 14. The base 12 may thus be considered to be the more conventional actuation surface of the device.

There is a mechanical coupling of the cover part 14 to the base so that the actuation can be transferred from the actuation action to the base and also to make sure the replaceable part cannot easily come off.

The cover part 14 is part of a system for sterilizing the actuation surface, namely the surface which is for contact by a user to implement an actuation.

Rotation of the cover part around the length axis of the handle may be inhibited or permitted. It might be a feature, such as using a rotation to form an electrical connection to turn the sterilization feature on and off. Different rotational positions may be used to make different electrical contacts, for example to choose a higher or lower sensitivity. Different rotational positions may for example allow in-coupling of more or different sterilization light sources.

The cover part 14 is in particular part of a first portion 16 of the sterilizing system. This first portion 16 is removable from the base 12 for replacement. There is a second portion 18 of the sterilizing system which is intended for continued use when the first portion 16 is replaced.

The parts in the first portion 16 may be low cost and designed for a relatively short lifespan whereas the parts in the second portion 18 are designed for a longer lifespan.

The sterilizing system 16,18 includes a light source 20 for providing sterilizing light to the actuation surface via the waveguide formed by the cover part 14, a detector 22 for detecting touching of the actuation surface and a controller 30 for controlling the light source in dependence on detected touching. The light source for example comprises a UV light source such as a UV-C LED or set of UV-C LEDs. It is known that UV light can be used to perform a sterilizing function.

The waveguide formed by the cover part 14 transmits the sterilizing light from the light source 20 to the actuation surface. Light is out-coupled from the waveguide at the actuation surface and thereby provides sterilization of the actuation surface when it passes through. The cover part is designed to be resistant to wear and tear and to be easily replaced by a user.

The amount of out-coupling of light depends at least in part on the surface properties of the waveguide. In particular, light will escape if the conditions for total internal reflection are not met. This may for example be achieved by incorporating scattering particles in the waveguide so that some of the light will be scattered to have an angle that allows out-coupling. Alternatively, a surface pattern may be used which defines light out-coupling features. The design of the light out-coupling features can be optimized to ensure a uniform out-coupling of light from all of the actuation surface or desired contact areas of the actuation surface. This is a more direct way of coupling light out from the waveguide.

The design may be such that there is very little, if any, out-coupling in case of a new and hence clean door handle. The light may escape when and where there is biological contamination present on the surface. A small amount of base radiation escaping from the light guide provides a tolerance margin.

If the handle is touched or contacted by any solid material (hence having a different refractive index to air), the out-coupling characteristics change, and the resulting difference in the light passage along the waveguide can be detected at the detector 22. Thus, the controller can determine from the signal at the detector 22 when the handle is being touched. The controller for example detects touching by detecting an increase in light out-coupled from the waveguide and hence a decrease in light reaching the detector. The touching alters the refractive index boundary conditions and thus allows more light to escape from the waveguide. Some light may be scattered back into the light guide as a result of the contact, but there will generally be an overall change in light characteristics. In theory, the amount of extra out-coupling could be exactly balanced by the amount of light that is reflected back into the light guide, but this can be ruled out as an extremely unlikely eventuality.

The controller may then turn off the light source during detected touching. This reduces the exposure of a person to the generated UV radiation. The light source may then be turned on after the detected touching has ended to resume the sterilizing function. The controller may also detect contamination of the actuation surface by detecting an increase in light out-coupled from the waveguide, but by an amount less than when the surface is touched. The light source may remain turned on in response to detected contamination. In this way, the light source remains activated if out-coupling is increased due to surface contamination.

The controller may also detect a level of wear and tear of the cover part. For example, scratches may be detected as they also cause an increase in light out-coupling. Thus, both the level of light out-coupling (and hence the corresponding change in the detected light) and the temporal characteristics may be used to identify different surface conditions of the actuation surface.

The controller can then provide an output to indicate when the first portion should be replaced. This output may for example via e.g. a visible LED indicator (green/red). This ensures that the touch sensing function remains reliable, by changing the first portion.

When a new cover part is installed, it may have different optical properties to the previous cover part. The controller may thus perform a recalibration to adapt to a new cover part that has just been installed. This recalibration may also be used if the surface is cleaned physically (rather than optically) as this will remove deactivated bacteria and may thus also change the light out-coupling properties.

The example above makes use of optical detection of touching. The detector may also be used as a safety sensor to ensure the UV radiation is not too high e.g. as a result of a component failure of the light source. Any scenario where too much light is detected can be considered a safety risk (especially when the exact cause is unknown), and thus a decision to turn off the light is appropriate.

The detection of touching by monitoring the light level requires the light source to be initially turned on. The light source may be turned on permanently (apart from during detected touch events) so that the detection function is always active.

When the light source is turned off, detection based on light detection is no longer possible. For this purpose, it is necessary to use an alternative way to determine when exactly the touch event has ended.

One option is to probing the light source with very short (∼ ms) pulses of UV light to allow intermittent detection to take place, or a visible light source (which is not harmful) may be used for the detection when the UV light source is deactivated.

However, to save power, the light source may only be turned on for a duration required to implement the sterilization, after a touch event. In this case the initial detection of the touch event cannot rely on detection of light from the light source, since it may be turned off.

In this case, touching may be detected based on the actuation itself. For example, a door handle may include a sensor for sensing actuation of the handle (e.g. a switch contact is made when the handle is turned) and this may be used to inform the controller that the door handle has been touched. The sterilization light may then be provided for a certain (pre-determined) time after the door handle has returned to the non-actuated position.

Various other algorithms could be applied for the on/ off decision for example based on the trade-off between power saving and disinfection rate (since e.g. 10mWh will provide for 90% disinfection, and 20mWh thus provides for 99%, etc.). A quick, short, high power disinfection right after a touch event might even be followed by a low power, permanent, maintenance dose.

There are various possible divisions of the components between the first and second portions 16, 18, discussed below. However, the first portion 16 is primarily an optical part and it is preferably disposable and can easily be replaced.

Fig. 1 shows the detector 22 as part of the first portion, but the first portion may instead be an entirely passive optical part. If the detector and light source are both in the second (fixed) part, the first (disposable) part may for example include a mirror at the end to reflect light to the detector.

The second portion 18 may be considered to be an active part, that includes at least some of the electronic or active optical components, such as the optical source 20, detector 22, and driving electronics. In this way, when the actuation surface becomes damaged, so that the detection function is no longer reliable, it can be replaced simply and with low cost.

Figs. 2 to 5 show various possible implementations.

Fig. 2 shows the arrangement of components of Fig. 1. The first portion 16 comprises the cover part 14 and also the detector. The second portion 18 comprises the light source 20, the controller 30 and a power source, in particular a battery 32. The cover part 14 includes wiring 40 to the detector 22. This wiring connects for example to the controller, which then has connections within the second portion 18 to the battery and light source.

In this design, there needs to be an optical, mechanical and electrical interface between the first and second portions 16,18. The optical interface may simply be that the light source emits light in a primary direction perpendicular to an end light emission face of the second (fixed) portion 18. The light then enters the waveguide formed by the cover part at a light entry face. These two faces for example butt together face-to-face to provide a normally directed light path across the interface.

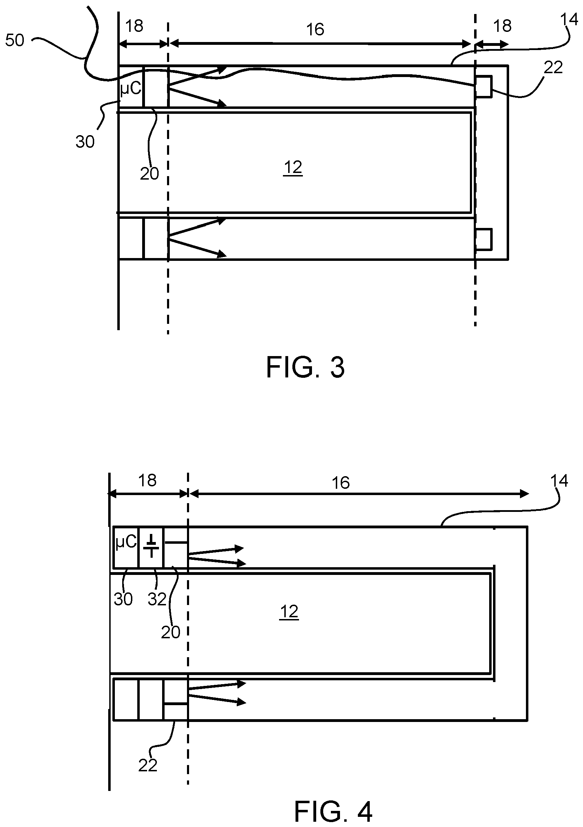

Fig. 3 shows an alternative arrangement of components. In this case, the power is provided from a wired connection 50 to an external power source. This is possible for fixed installations, and is for example possible for door handles (with power routed to the door through a suitable hinge arrangement).

The detector is again at the remote end of the handle, but it is in this example part of the second portion. The second portion 18 thus comprises the light source 20, the detector 22 and the controller 30. The part of the second portion which includes the detector may be removed to allow removal of the cover part, which is then the only component of the first portion 16. The first portion is thus entirely passive. In this design, there need to be two optical, mechanical and electrical interfaces between the first and (two) second portions.

As an alternative, the replaceable part may be flexible (e.g. silicone) so it can be wrapped around the handle. In this case, it is not required to remove the end of the second portion 18. In this design, the electrical connections may be fixed, so that only optical connections need to be made to the replaceable part.

Fig. 4 shows an alternative arrangement of components. In this case, the detector 22 is at the same end as the light source 20, the controller 30 and power source 32. The light reaching the detector still depends on the amount of light out-coupling and a mirror may be used to direct light towards the detector. The mirror may be at the distal end of the cover part 14 to make sure at least some of the light will reach the detector. The cover part 14 is again the only component of the first portion 16. The first portion is thus entirely passive.

In this design, there needs to be only an optical and mechanical interface between the first and second portions 16,18.

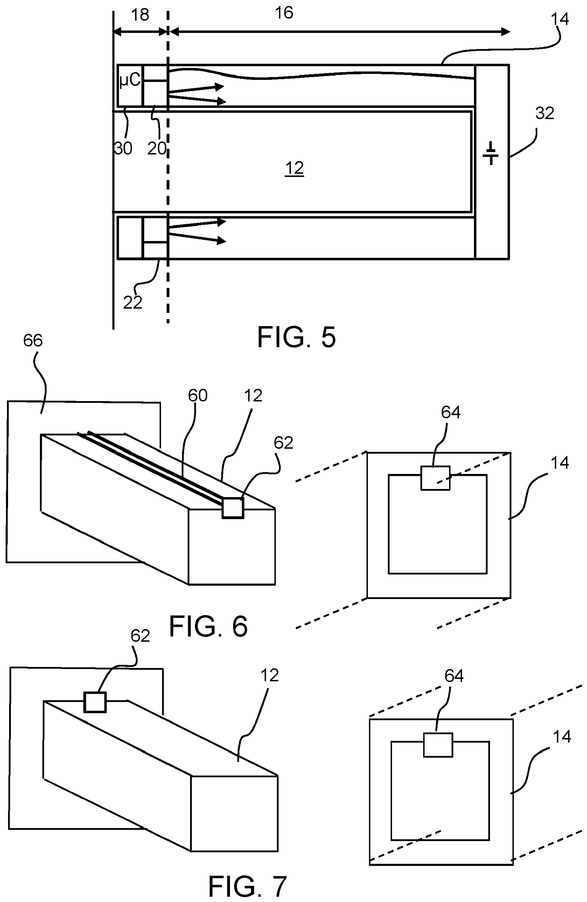

Fig. 5 shows an alternative arrangement of components. As in Fig. 4, the detector 22 is at the same end as the light source 20, and the controller 30. The power source 32 is part of the first portion 16 and is thus replaced with the cover part. The power source may for example have a similar lifespan to the cover part, so they can be replaced together by a single replacement operation.

In this design, there needs to be an optical, mechanical and electrical interface between the first and second portions 16,18.

As explained above, there is an interface between the first and second portions and in some examples this interface needs an electrical coupling to route power from the battery to the light source and controller, an optical connection to couple light from the light source in the second portion to the cover part, and a mechanical coupling to transfer force reliably from the cover part to the base. The electrical connection requires at least two leads.

Typically, the light source and electronics, being the expensive parts and the parts with the longest lifetime, will reside inside the second (active) part and the first portion will contain the other parts, such as a battery and the cover part which defines the active optical surface.

In the example shown, the mechanical coupling between the cover part and the base is provided by an interference fit, for example with a square cylindrical base and the cover part in the form of a sleeve with a corresponding square cross section. A fixing may be provided to retain the cover part in position, for example a grub screw or other latch system that can releasable fix the cover part to the base.

More generally, the cover part for example has a polygonal central bore for fitting over a corresponding polygonal outer shape of the shaft, thereby to implement a mechanical coupling. The polygonal central bore is for example square or rectangular. A rotationally symmetric shape may be used so that there are multiple possible fitting orientations or a non-rotationally symmetric shape may be used so that the cover part can be fitted in only one, correct, orientation.

Fig. 6 shows a first way to implement the electrical and optical connection. The base 12 is provided with two wires 60 which extend along the shaft formed by the base and terminate at a terminal block 62 (of one or more first terminals) at a distal end of the shaft. The sleeve formed by the cover part 14 has a closed end at which a second terminal block 64 is provided (of one or more second terminals) for coupling to the first terminal block. When the sleeve is fully fitted over the shaft the terminal blocks engage but also the end face of the sleeve makes optical connection with an optical output face 66 such that light delivered by the light source is coupled from the output face 66 to the sleeve.

There may be a single light source and the waveguide may distribute the light around the cover part, but there may instead be multiple light sources, for example arranged around the output face 66. The terminal blocks engage by a push fit coupling.

In one example, the cover part 14 houses the battery, and the wires 60 are power lines to the remaining parts of the sterilizing system, such as the light source, detector and controller. The light source can only light up if the cover part is slid completely onto the fixed base. This provides a safety feature in that the light source is not able to be turned on when the optical part is not fully connected. Thus it is ensured that the light is captured inside the light guide. Similarly, only when the optical end face of the cover part (the light entry surface) is slid far enough onto the fixed base, to form a face-to-face coupling with a light emission surface of the light source in the base, will the required light coupling take place from the light source into the sleeve.

Fig. 7 shows a second way to implement the electrical and optical connection. The first terminal block 62 (of one or more first terminals) is provided at a proximal end of the shaft (i.e. the end of the door handle nearest the hub). The second terminal block 64 (of one or more second terminals) is provided at distal end of the shaft (i.e. the free end of the door handle), for coupling to the first terminals. When the sleeve is fully fitted over the shaft the terminal blocks engage and again the end face of the sleeve makes optical connection with an optical output face 66 such that light delivered by the light source is coupled to the sleeve.

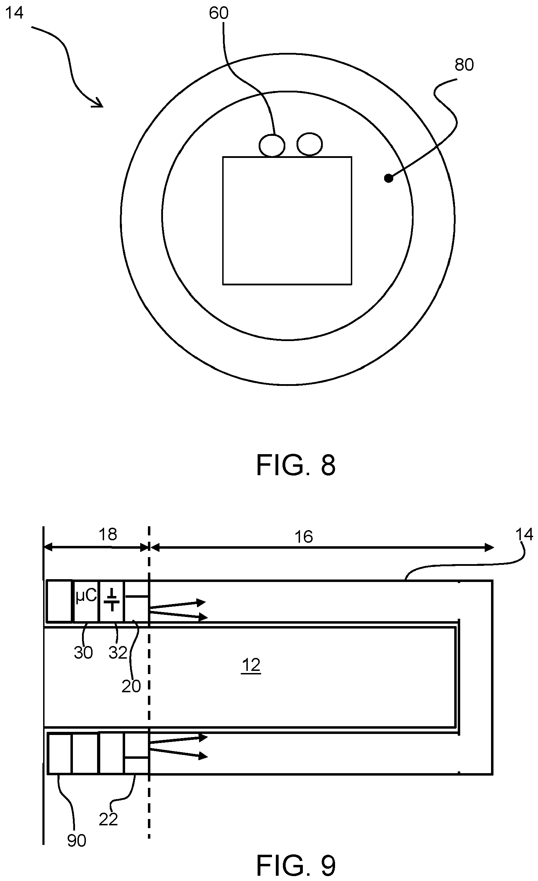

Fig. 8 shows another alternative design in which the battery 80 is defined in a space between the shaft formed by the base 12 and the cover part 14. The battery 80 has a hollow core which defines a central bore of the sleeve formed by the cover part 14. The battery is then part of the replaceable unit.

Alternatively, the battery can be provided in a hollow portion of the shaft. For example, the base 12 may be a hollow cylinder e.g. of steel which provides a space for a standard cylindrical battery.

The system may be for sterilizing door handles on both sides of a door. In such a case, an electrical connection may be provided between the two fixed (second) parts 18 for passing through the door. There may then be only one controller and/or one battery for mounting at the side of only one door handle. Power may then be routed from one side to the other side, for example for powering a light source and/or detector on the opposite side of the door. Feedback signals as well as power may be routed through the door.

For example, a UVC sensor may be used for safety reasons as mentioned above, and feedback paths for the sensor signal back to a single controller may be provided.

The electrical connection for example comprises two or more wires, which may be used for providing power to an LED on the other side of the door, and/or for providing a path for a sensor signal from the other part of the door.

Similarly, the light source may be for mounting at the side of only one door handle, and the system then further comprises an optical connection, such as an optical fiber, between the first and second first portions for passing through the door. A single LED or LED arrangement may for example be used for both sides.

Optical feedback signals may also be routed using this optical connection. Thus, an optical connection may be used for the disinfection light, but also for a possible optical sensor signal.

Thus, in each case, the part of the door handle that connects through the door (which attaches both door handles on each side of the door to each other) also implements an electrical connection and/or an optical connection. These connections are of interest because some parts are only needed once, such as the battery and light source (if there is an optical path through the door), whereas some other parts are needed on both sides such as the cover part and sensor.

Some parts, such as the battery could be placed inside the door. Power may for example be provided using a modified spindle that connects the door handles on each side of the door.

In the examples above, there is a battery power supply or else a connection to an external power supply.

Fig. 9 shows an alternative example in which energy is harvested from the normal manual actuation. The system of Fig. 9 corresponds to that of Fig. 4, with all electrical parts in the fixed (second) part 18. However, the energy harvesting may be applied to the other examples, with a different division of parts between the removable portion and the fixed portion. The energy harvesting may also be applied as an overall system without the division into fixed and replaceable parts as described above.

Fig. 9 shows an energy harvesting circuit 90 which is used to charge the battery 32. The actuator may be used in its normal way, but while doing so, a small amount of energy is harvested. The energy is subsequently used to power the light source 20 (and optionally also a detector). The light source will in turn disinfect the surface that was touched while using the actuator. This forms a closed loop control system which ensures that the actuation surface will always be disinfected after use (because energy is then generated), and it will not run out of power. The battery is only needed for temporary storage of energy harvested and it does not require a mains connection.

The energy storage may be a capacitor or super capacitor instead of a battery.

The example of Fig. 9 is a door handle (or door knob) as in the examples above. In some countries, rotary knobs are common and in others pivoting levers are common. In all cases, energy can be harvested from this movement, by adding a small electricity generator, similar to a dynamo lamp generator.

Energy harvesting circuits are well known. Typically, they comprise a magnet which is actuated to move through an electrical coil (or vice versa), or a magnet which is rotated between coils. The energy harvesting can be implemented by adding a magnet and electrical coil to the different parts (fixed and movable) of the door handle. This provides energy by inducing an EMF onto coils.

Piezoelectric materials may also be used to generate energy in response to mechanical deformation, based on converting mechanical strain into voltage or current.

The generation of electrical energy by these techniques will be well known to those skilled in the art.

There are numerous examples in addition to the integration into a door handle or knob. Opening a door by pushing against the door (at a designated contact area) enables relative movement between parts to be implemented at the hinges, which can then thus be equipped with the energy harvesting circuit. The contact area can then be sterilized.

A toilet seat is opened and closed so again the hinges can be equipped with an energy harvesting circuit to power a sterilizing system for the part of the toilet seat that is touched. Alternatively, a closed toilet seat may be seated on springs, which will be compressed when sitting down. The weight of the body is then used to generate power.

The energy harvesting may be applied to buttons which are pushed, such as elevator buttons or buttons for other applications for example where mains power is not available.

The amount of power that may be harvested from a small movement, such as pushing a button or turning a knob, is obviously relatively small. However, the amount of power needed to disinfect a small surface is also low, given the size of the typical button or other manual contact area.

The energy harvesting may be applied to exercise equipment, wherein lifting, pressing, rotating, or pushing heavy weights is a common element of exercise equipment; and energy can easily be harvested.

There are many other possible examples including hand tools (a bicycle pump or a screwdriver) or other handles (handlebars of a bike, or a safety bar in a roller coaster).

In some examples, the cover part may comprise a sleeve for a handle, but for buttons (e.g. elevator buttons), the cover part could be in the form of a cover disc or patch. For ATMs or other screen type keypad devices, the cover part could be a waveguide foil that is applied to the surface; comparable to a phone screen protector.

The sterilizing light may be provided continuously in some examples, possibly interrupted only when there is physical contact to reduce the exposure to the contacting person. In other examples, the manual actuation may be detected and the sterilization takes place after the actuation to sterilize the surface that has been contacted. This applies to the example using energy harvesting, in that the generation of energy serves also as a means for sensing that there has been use of the actuator.

As mentioned above, other sensors may be used to detect that there has been manual actuation so that the light can be provided as soon as the actuation has ended for a given period of time to implement the sterilization. This may be used as well as (or instead of) monitoring a level of light leakage in order to detect contact with the actuation surface.

Some examples above implement the sterilizing system with a first portion 16 which is removable from a base for replacement, including at least the cover part 14, and a second portion 18 which is intended for continued use when the first portion 16 is replaced. However, in other examples, the system is implemented as a single unit for application to a base. In such as case, the system comprises a cover part 14 including the actuation surface and for transferring actuation to the base through the cover part, a light source 20 for providing sterilizing light to the actuation surface and a controller 30 for controlling the light source. The cover part 14 comprises a waveguide which is transparent to the sterilizing light, thereby to transmit the sterilizing light from the light source to the actuation surface.

The invention is of particular interest for devices used and shared in public spaces, such as a public door knob (e.g. of a restaurant or public toilet etc.).

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A system (16,18) for sterilizing an actuation surface which is for contact by a user to implement an actuation, the system comprising:
a cover part (14) including the actuation surface, the cover part being for fitting to a base (12), the cover part (14) being for transferring actuation to the base through the cover part;
a light source (20) for providing sterilizing light to the actuation surface; and
a controller (30) for controlling the light source,
wherein the cover part (14) comprises a waveguide which is transparent to the sterilizing light, thereby to transmit the sterilizing light from the light source to the actuation surface,
wherein the system comprises a first portion (16) which is removable from the base for replacement and includes at least the cover part (14), and a second portion (18) which is intended for continued use when the first portion is replaced, and
wherein the system comprises a coupling between the first and second portions (16,18) which implements an electrical and optical coupling.

2. The system of claim 1, further comprising a detector (22) for detecting touching of the actuation surface, wherein the controller (30) is for controlling the light source in dependence on detected touching.

3. The system of claim 2, wherein the detector (22) comprises a light detector, and the controller (30) is adapted to detect touching by detecting a change in light out-coupled from the waveguide.

4. The system of claim 2 or 3, wherein the controller (30) is adapted to turn off the light source during detected touching.

5. The system of any one of claims 1 to 4, wherein the optical coupling comprises face-to-face contact between a light entry surface of the waveguide and a light emission surface of the light source (20) only when the first portion is fully fitted to the base.

6. The system of any one of claims 1 to 5, wherein the first portion (16) has a polygonal central bore for fitting over a corresponding polygonal outer shape of the base (12), thereby to implement a mechanical coupling.

7. The system of claim 6, wherein the polygonal central bore is square or rectangular.

8. The system of any one of claims 1 to 7, further comprising one or more wires (60) extending along the base and terminating at one or more first terminals (62) at a distal end of the base, and the first portion (16) has a closed end including one or more second terminals (64) for coupling to the first terminals, thereby to implement the electrical coupling when the first portion (16) is fully fitted to the base (12).

9. The system of any one of claims 1 to 8, further comprising one or more first terminals (62) at a proximal end of the base (12), and the first portion (16) comprises one or more second terminals (64) at a distal end for coupling to the first terminals (62).

10. The system of any one of claims 1 to 9, wherein the first portion (16) comprises a battery (32;80), wherein the battery is:
in a space between the base (12) and the cover part (14), wherein the battery has a hollow core which defines a central bore of the first portion; or
in a hollow part of the base.

11. The system of any one of claims 1 to 10, comprising first and second cover parts of respective first and second first portions, for sterilizing a pair of door handles, wherein the system comprises an electrical connection between the first and second first portions for passing through a door.

12. The system of claim 11, comprising only one controller and/or only one battery for mounting at the side of only one door handle.

13. The system of claim 11 or 12, wherein the light source is for mounting at the side of only one door handle, and the system further comprises an optical connection between the first and second first portions for passing through the door.

14. The system of any one of claims 1 to 12, wherein the light source (20) comprises a UV light source.

15. A door handle (10), comprising:
a base (12); and
the system of any one of claims 1 to 14 defining an actuation surface for transferring actuation to the base through the cover part.
